# EUROPEAN PATENT APPLICATION

(11) **EP 1 520 578 A1**
(43) Date of publication of application: **06.04.2005**
(21) Application number: 04256049.0
(22) Date of filing: 30.09.2004
(51) Int. Cl.: A61K 9/10, A61K 47/18

(54) **Medicinal suspensions**

(30) Priority: 30.09.2003 US 674702
(71) Applicant: McNEIL-PPC, Inc., Skillman, New Jersey 08558 (US)
(72) Inventor: Buchler, Gail K., Lower Gwynedd, PA 19002 (US); Shapiro, Kenneth B., Lawrenceville, New Jersey 08648 (US); Osei, Anthony A., Harleysville, PA 19438 (US); Shapiro, Kenneth B., Lawrenceville, New Jersey 08648 (US)
(74) Representative: Tunstall, Christopher Stephen

(57) **Abstract**

The present invention relates to a pharmaceutical suspension having improved pH and viscosity and particle size stability and stable uniform distribution of active ingredient. The suspensions contain a therapeutically effective amount of suspended solid particles comprising pharmaceutical active ingredient, a thickening component, and an amino polycarboxylic acid compound and in certain embodiments, a nucleation inhibitor as a means to maintain a stable uniform suspension product. The invention further relates to their method of manufacture and use.

## Description

### FIELD OF INVENTION

The present invention relates to aqueous suspensions having at least one pharmaceutical active ingredient and suspending system having a thickening component, at least one amino polycarboxylic acid compound and optionally a nucleation inhibitor in which the resulting suspensions exhibit improved pH and viscosity stability and uniform suspension product.

### BACKGROUND

Orally administered medicaments (pharmaceutical active ingredients) are given to the patient in many forms, including solid form such as capsules or tablets, and liquid form such as solutions, emulsions or suspensions.

Children, older persons, and many other persons including disabled or incapacitated patients have trouble swallowing whole tablets and capsules. Therefore it is desirable to provide the medicine either in a chewable or orally disintegratable solid form or a liquid form. For many patients, including pediatric and geriatric patients, a liquid oral dosage form is preferable over chewable dosage form because of the ready swallowability without chewing of the liquid dosage form.

A common problem associated with liquid dosage forms is the often disagreeable taste of the active ingredient or active ingredients that manifests itself during the time that the liquid dosage form is in the mouth prior to swallowing, and the aftertaste from residual active ingredient remaining in the oral cavity after swallowing. In some cases, adding flavoring ingredients to the liquid that can cover or mask the bitter or unpleasant taste of the active ingredient may conceal the taste of the active ingredient in a liquid form. For instance, this approach was employed with a first generation pediatric liquid dosage form of acetaminophen (N-acetyl para-aminophenol or "APAP"). APAP was available commercially through the 1980s in an aqueous solution that included flavor ingredients employed to mask the unpleasant taste of the APAP. However, these agents are not totally effective in concealing the unpalatable taste of most pharmaceutical active ingredients.

Aqueous suspension formulations were developed in the late 1980s/early 1990s to provide improved tastemasking ability for slightly or poorly soluble active ingredients such as APAP, that can be rendered relatively insoluble by adjusting conditions such as pH, ionic strength, and free water content of the vehicle. Minimizing the amount of unpleasant tasting active ingredient in the solution state provided a substantial decrease in the level of perception of the unpleasant taste during the short residence of the suspension in the oral cavity prior to swallowing, and also helped to minimize aftertaste due to residual dissolved active ingredient remaining in the oral cavity after swallowing. These formulations overcame many basic challenges of preparing undissolved pharmaceutical actives in storage stable ready-to-use liquid dosage form. Formulations were developed that eliminated the problems of separation or settling out of the undissolved ingredients. Additionally, the suspension dosage forms eliminated the need for many undesirable cosolvents such as ethanol, and propylene glycol, which can both impart a stinging sensation when used above certain levels.

U.S. Patent No. 5,759,579 describes liquid suspensions for pharmaceutically active ingredients. The suspension systems comprise water and, as the suspending agents, xanthan gum and hydroxypropyl methylcellulose.

U.S. Patent No. 5,621,005 describes aqueous pharmaceutical suspension compositions comprising substantially water insoluble pharmaceutical actives, suspension agents, and taste-making agents. The compositions contain a suspension stabilizing effective amount of xanthan gum, pregelatinized starch and polyoxyethylene sorbitan monooleate.

U.S. Patent No. 5,658,919 describes an aqueous pharmaceutical suspension containing suspended acetaminophen and at least one additional pharmaceutical active, a suspension system containing xanthan gum, a mixture of microcrystalline cellulose and sodium carboxymethyl cellulose and an auxiliary suspending agent.

U.S. Patent No. 6,132,758 describes antihistamine syrups using one or more aminopolycarboxylic acid compounds.

U.S. Patent No. 5,183,829 describes oral compositions prepared by adding selected dispersing agents such as a polyvinylpyrrolidone, hydroxypropyl methylcellulose or hydroxypropyl cellulose to non-steroidal anti-inflammatory drugs in a medium of polyol-glycol-alcohol.

The present invention is directed to discovery of a stable aqueous, preferably acidic, suspension system for substantially water insoluble pharmaceutical actives, which when combined with selected amino polycarboxylic acid compounds, exhibits improved pH and viscosity stability. This invention also provides for the inclusion of a nucleation inhibitor and a method for incorporating a hydrophobic drug substance uniformly into the stable suspension product.

### SUMMARY OF THE INVENTION

As embodied and fully described herein the present invention provides an aqueous pharmaceutical suspension composition comprising a solid pharmaceutical active particle, a particle suspending effective amount of at least one thickening agent, a suspension stabilizing effective amount of at least one amino polycarboxylic acid compound or salt thereof and optionally a nucleation inhibitor. The pharmaceutical active is preferably hydrophobic and substantially insoluble in an aqueous acidic solution that is preferably alcohol free.

For purposes of this invention, a suspension means a liquid system having solid particles dispersed substantially throughout. A suspension does not encompass emulsions, which are meant to describe liquids suspended within liquid carriers or syrup formulations containing substantially fully dissolved pharmaceutical actives. As used herein, a "particle" may be a crystal, a granule, an agglomerate, or any undissolved solid material. The particles of the present invention preferably have a median particle size (d50%) of from about 5 to about 200 microns, more preferably from about 5 to about 100 microns, most preferably from about 5 to about 11 microns.

In one embodiment, the invention is a suspension comprising a blended thickening component having a structuring agent, such as xanthan gum and a swelling agent, such as pregelatinized starch, and a surfactant, such as polyoxyethylene sorbitan monooleate, and an amino polycarboxylic acid or salt thereof, such as ethylenediaminetetraacetic acid (EDTA), and optionally a nucleation inhibitor, such as polyvinylpyrrolidone and a substantially water insoluble pharmaceutical active. In a further embodiment, the suspension further comprises a taste modifying component having from about 20 to 50% sucrose, from about 0 to 20% sorbitol weight by volume of the total suspension. Weight per unit volume is based on a metric relationship, such as, for example, grams per milliliter or kilograms per liter. All units herein are percent weight per unit volume unless otherwise indicated.

As embodied and fully described herein the present invention also provides a process for preparing an aqueous pharmaceutical suspension composition comprising the steps of:
(a) high shear premixing from about 0.05 to 40% of the substantially water insoluble pharmaceutical active with from about 0 to 0.1% of a surfactant, such as polyoxyethylene sorbitan monooleate and from about 0 to 0.1% of a defoamer, such as a 30% simethicone emulsion;
(b) separately dispersing at least one thickener, such as xanthan gum and/or pregelatinized starch, optionally with a nucleation inhibitor, such as polyvinylpyrrolidone in about 50% water until uniformly dispersed;
(c) adding and mixing the active premix of step (a) with the aqueous mixture of step (b);
(d) adding from about 0 to 20% of a polyhydric alcohol sweetener, preferably sorbitol, 20 to 50% sugar, preferably sucrose, to the dispersion of step (c) and mixing until the ingredients are uniformly dispersed in the mixture;
(e) adding at least one selected preservative;
(f) adding at least one selected amino polycarboxylic acid compound and mixing to dissolution;
(g) admixing sufficient pharmaceutically acceptable pH adjuster, such as citric acid for acidity or sodium carbonate for alkalinity, to target the pH of the final solution to between about 3.7 to 8.0 to the mixture of step (f) until the ingredients are uniformly dispersed throughout the mixture;
(h) adding colorants, sweeteners, flavors, and
(i) adding and mixing sufficient water to the mixture of step (h) to produce an aqueous pharmaceutical suspension of 100% desired volume.

Another embodiment of the invention involves dry blending of a hydrophobic active with carrier in place of a high shear premix. The dry blend embodiment begins by dispersing the hydrophobic active preblend in water that contains a surfactant and ensures complete uniformity of the active in the suspension product.

### BRIEF DESCRIPTION OF THE DRAWINGS

Figure 1 is a graph that illustrates stabilization effects for suspension viscosity.

Figure 2 is a graph that illustrates the effects of EDTA addition on pH stabilization.

### DETAILED DESCRIPTION OF THE INVENTION

In a pharmaceutical suspension, typically at least one active ingredient is present substantially in the form of undissolved solid particles. However, in any such system, at least a small portion of the active ingredient will be in the dissolved state. In formulating such systems, it is advantageous to minimize the amount of drug present in the dissolved state. Minimizing the amount of active ingredient in solution is advantageous for both the taste and chemical stability of the product. Aqueous suspension oral dosage forms provide an alternate means to tablets, caplets, and capsules for oral dosage that are advantageously more easily swallowed.

In certain embodiments of the invention, the suspended particles contain active ingredient. These are referred to herein as "active particles" or "active ingredient particles". In one embodiment, the suspended particles are substantially pure crystals of the active ingredient having a median particle size (d50%) from about 5 to about 11 microns. In another embodiment, the suspended particles are agglomerates, e.g. granules, comprising active ingredient. In another embodiment, the suspended particles further comprise a coating on their surface, e.g. a polymer coating for the purpose of tastemasking or modified release. Suitable particle coating systems for tastemasking are known in the art.

Examples of suitable taste masking coatings for particles are described in U.S. Patent No. 4,851,226, U.S. Patent No. 5,075,114, and U.S. Patent No. 5,489,436. Commercially available taste masked active ingredients may also be employed. For example, acetaminophen particles that are encapsulated with ethylcellulose or other polymers by a coacervation process may be used in the present invention. Coacervation-encapsulated acetaminophen may be purchased commercially from Eurand America, Inc. (Vandalia, Ohio) or from Circa Inc. (Dayton, Ohio). Examples of suitable release modifying coatings for particles are described in U.S. Patent Nos. 4,173,626; 4,863,742; 4,980,170; 4,984,240; 5,286,497; 5,912,013; 6,270,805; and 6,322,819.

Loratadine is a particularly preferred substantially water insoluble pharmaceutical active ingredient useful in accordance with the invention. Other preferred substantially water insoluble pharmaceutical active ingredients useful in accordance with the invention are listed below. For the purposes of the present invention, the term substantially water insoluble includes compounds that are insoluble, practically insoluble or only slightly soluble in water as defined by U.S. Pharmacopeia, 24^{th} edition, and also compounds that are insoluble, practically insoluble or slightly soluble in aqueous solution at pH 3.0 to 6.9. Given the strong pH dependence of loratadine, pH stability is a very important requirement for loratadine liquid suspensions. This invention is particularly advantageous for use with liquid suspensions having similar pH dependence characteristics.

Suitable active ingredients for use in this invention include for example pharmaceuticals, minerals, vitamins and other nutraceuticals, oral care agents, flavorants and mixtures thereof. Suitable pharmaceuticals include analgesics, anti-inflammatory agents, antiarthritics, anesthetics, antihistamines, antitussives, antibiotics, anti-infective agents, antivirals, anticoagulants, antidepressants, antidiabetic agents, antiemetics, antiflatulents, antifungals, antispasmodics, appetite suppressants, bronchodilators, cardiovascular agents, central nervous system agents, central nervous system stimulants, decongestants, diuretics, expectorants, gastrointestinal agents, migraine preparations, motion sickness products, mucolytics, muscle relaxants, osteoporosis preparations, polydimethylsiloxanes, respiratory agents, sleep-aids, urinary tract agents and mixtures thereof.

Suitable oral care agents include breath fresheners, tooth whiteners, antimicrobial agents, tooth mineralizers, tooth decay inhibitors, topical anesthetics, mucoprotectants, and the like.

The invention will now be described specifically in terms of the preparation of aqueous suspensions of loratadine. Loratadine is a medicament (pharmaceutical active ingredient) used as an antihistamine. Loratadine is the drug name given to the compound known as ethyl 4-(8-chloro-5,6-dihydro-11H-benzo[5,6]cyclohepta[1,2-b]pyridin-11-ylidene) -1-piperidinecarboxylate and having the empirical formula C₂₂H₂₃ClN₂O₂. The compound descarboethoxyloratadine is an antihistaminic active metabolite of loratadine. A closely related antihistamine is azatadine. Reference will also be made in detail herein to other preferred embodiments of the compositions, processes and methods of the invention.

Suitable flavorants include menthol, peppermint, mint flavors, fruit flavors, chocolate, vanilla, bubblegum flavors, coffee flavors, liqueur flavors and combinations and the like.

Examples of suitable gastrointestinal agents include antacids such as calcium carbonate, magnesium hydroxide, magnesium oxide, magnesium carbonate, aluminum hydroxide, sodium bicarbonate, dihydroxyaluminum sodium carbonate; stimulant laxatives, such as bisacodyl, cascara sagrada, danthron, senna, phenolphthalein, aloe, castor oil, ricinoleic acid, and dehydrocholic acid, and mixtures thereof; H2 receptor antagonists, such as famotadine, ranitidine, cimetidine, nizatidine; proton pump inhibitors such as omeprazole or lansoprazole; gastrointestinal cytoprotectives, such as sucralfate and misoprostol; gastrointestinal prokinetics, such as prucalopride, antibiotics for Hpylori, such as clarithromycin, amoxicillin, tetracycline, and metronidazole; antidiarrheals, such as diphenoxylate and loperamide; glycopyrrolate; antiemetics, such as ondansetron, analgesics, such as mesalamine.

In one embodiment of the invention, the active ingredient may be selected from bisacodyl, famotadine, ranitidine, cimetidine, prucalopride, diphenoxylate, loperamide, lactase, mesalamine, bismuth, antacids, and pharmaceutically acceptable salts, esters, isomers, and mixtures thereof.

In another embodiment, the active ingredient is selected from analgesics, antiinflammatories, and antipyretics: e.g. non-steroidal anti-inflammatory drugs (NSAIDs), including propionic acid derivatives: e.g. ibuprofen, naproxen, ketoprofen and the like; acetic acid derivatives: e.g. indomethacin, diclofenac, sulindac, tolmetin, and the like; fenamic acid derivatives: e.g. mefanamic acid, meclofenamic acid, flufenamic acid, and the like; biphenylcarbodylic acid derivatives: e.g. diflunisal, flufenisal, and the like; and oxicams: e.g. piroxicam, sudoxicam, isoxicam, meloxicam, and the like. In a particularly preferred embodiment, the active ingredient is selected from propionic acid derivative NSAID: e.g. ibuprofen, naproxen, flurbiprofen, fenbufen, fenoprofen, indoprofen, ketoprofen, fluprofen, pirprofen, carprofen, oxaprozin, pranoprofen, suprofen, and pharmaceutically acceptable salts, derivatives, and combinations thereof. In another embodiment of the invention, the active ingredient may be selected from acetaminophen, acetyl salicylic acid, ibuprofen, naproxen, ketoprofen, flurbiprofen, diclofenac, cyclobenzaprine, meloxicam, rofecoxib, celecoxib, and pharmaceutically acceptable salts, esters, isomers, and mixtures thereof.

In another embodiment of the invention, the active ingredient may be selected from pseudoephedrine, phenylpropanolamine, chlorpheniramine, dextromethorphan, diphenhydramine, astemizole, terfenadine, fexofenadine, loratadine, desloratadine, doxilamine, norastemizole, cetirizine, mixtures thereof and pharmaceutically acceptable salts, esters, isomers, and mixtures thereof. In a particular embodiment, the active ingredient may be selected from fexofenadine, loratadine, desloratadine, terfenadine, astemizole, norastemizole, cetirizine, and pharmaceutically acceptable salts, esters, isomers, and mixtures thereof.

Examples of suitable polydimethylsiloxanes, which include, but are not limited to dimethicone and simethicone, are those disclosed in United States Patent Nos. 4,906,478, 5,275,822, and 6,103,260. As used herein, the term "simethicone" refers to the broader class of polydimethylsiloxanes, including but not limited to simethicone and dimethicone.

Examples of other water insoluble pharmaceutical active ingredients that can be used in accordance with the invention include but are not limited to the following examples: analgesics, such as APAP and ibuprofen; cardiovascular drugs, e.g. cardiac glycosides, clofibrate and probucol; hypoglycemic drugs; sedatives/hypnotics, e.g. barbiturates, disulfiram and glutethimide; antiepileptics, e.g., carbamazepine, mephenytoin, phenytoin and phensuximide; psycholpharmacologic agents e.g. perphenazine; analgesic, antipyretic and anti-inflammatory agents, e.g. naproxen, oxycodone, indomethacin, and phenylbutazone; antineoplastic drugs such as lomustine; and antimicrobials such as erythromycin estolate.

The active ingredient or ingredients are present in a "unit dose volume" of the aqueous suspension in a therapeutically effective amount, which is an amount that produces the desired therapeutic response upon oral administration and can be readily determined by one skilled in the art. In determining such amounts, the particular active ingredient being administered, the bioavailability characteristics of the active ingredient, the dose regime, the age and weight of the patient, and other factors must be considered, as known in the art. As used herein a "unit dose volume" of the aqueous suspension is a convenient volume for dosing the product to a patient. The dosing directions instruct the patient to take amounts that are multiples of the unit dose volume depending on, e.g., the age or weight of the patient. Typically the unit dose volume of the suspension will contain an amount of active ingredient that is therapeutically effective for the smallest patient. For example, suitable unit dose volumes may include one teaspoonful (about 5 mL), one tablespoonful (about 15 mL), one dropper, or one milliliter.

In one embodiment, the aqueous pharmaceutical suspension composition in accordance with the present invention comprises from about 0.05% to about 40%, e.g. about 0.05 to about 0.2%, or about 1.6 to about 10%, or about 15 to about 40% weight per volume (w/v) of at least one active ingredient. Amounts of pharmaceutical active ingredient in this range are generally acceptable for taste modifying. It is possible that more than 40% of a water insoluble pharmaceutical active ingredient could be included in the suspension and be sufficiently taste masked for consumer acceptability. Suspensions containing less than 0.05% of pharmaceutical active ingredients are also possible.

In one embodiment, in which the active ingredient is loratadine, the level of active ingredient in the suspension is from about 2.5 to about 5 milligrams per teaspoonful, or from about 0.05 to about 0.2% w/v. In another embodiment, in which the active ingredient is acetaminophen, the level of active ingredient in the suspension is from about 80 to about 160 mg per teaspoonful, or about 1.6 to about 3.2% w/v.

In another embodiment, in which the active ingredient is acetaminophen, the level of active ingredient in the suspension is from about 80 to about 160 mg per 1.6 mL, or about 5 to about 10% w/v. In another embodiment, in which the active ingredient is ibuprofen, the level of active ingredient in the suspension is from about 50 to about 200 mg, e.g. about 100 mg per teaspoonful, or about 40 mg per 1 mL, or about 1 to about 4% w/v.

Stabilizing the suspension of water insoluble pharmaceutical active ingredients is a key component of the present invention. It has been found by the present inventors, that the storage stability of the suspension can be surprisingly enhanced by the addition of at least one selected amino polycarboxylic acid compounds. At least some such compounds, particularly EDTA, are known for use as chelating agents. EDTA has not been previously described as being suitable for improving pH and viscosity stability in liquid suspensions. It has additionally been found that the suspension can be further stabilized by the addition of a nucleation inhibitor that is believed to prevent the growth of particles. Further, in certain embodiments, the addition of a surfactant, such as polyoxyethylene sorbitan monooleate, produces homogeneously dispersed suspensions of water insoluble pharmaceutical active ingredients.

The suspensions of the present invention can employ suspending systems as known in the art that include at least one thickening agent. The thickening component typically comprises one or more thickening agents that may be selected from hydrophilic polymers such as hydrocolloids, swelling or gelling polymers, and the like. In one preferred embodiment, the thickening component combines the attributes of a structuring agent and a swelling agent.

A structuring agent, when introduced into an appropriate aqueous environment, forms an ordered structure, stabilized by hydrogen bonding and molecular entanglement. Hydrocolloids are a particularly good type of structuring agent. Hydrocolloids are dispersions of particles around which water molecules and solvated ions form a shell-like structure, fluid absorption occurs principally by swelling and enlargement of the structure.

Examples of suitable hydrocolloids include alginates, agar, guar gum, locust bean, carrageenan, tara, gum arabic, tragacanth, pectin, xanthan, gellan, maltodextrin, galactomannan, pusstulan, laminarin, scleroglucan, gum arabic, inulin, karaya, whelan, rhamsan, zooglan, methylan, chitin, cyclodextrin, chitosan, and combinations thereof. In certain embodiments of the present invention, xanthan gum is a preferred hydrocolloid for use as a structuring agent.

Xanthan gum is a high molecular weight natural carbohydrate, specifically, a polysaccharide. The xanthan gum suitable for use in the present invention is a high molecular weight polysaccharide produced by Xanthomonas campestris. Techniques and strains for producing this polysaccharide are described in U.S. Patent Nos. 4,752,580 and 3,485,719 (the disclosures of which are hereby incorporated by reference). The xanthan gum used in the present invention should have a viscosity in a one percent salt solution of from about 1000 to about 1700 cP (mPa-sec). The one percent solution's viscosity should be measured at 25 °C with an LV model Brookfield Synchro-Lectric viscometer at 60 rpm, no. 3 spindle. Xanthan gum is available from several commercial suppliers such a RT Vanderbilt Company and CP Kelco. Examples of suitable xanthan gums are Keltrol, Keltrol F, Keltrol T, Keltrol TF and Keltrol 1000 Keltrol, Keltrol TF and Keltrol 1000 are the xanthan gums for use in pharmaceutical suspensions.

A swelling agent, when exposed to an appropriate aqueous environment, expands without forming a network system. Pregelatinized starch is a particularly good swelling agent. Pregelatinized starch, also known as "instantized" starch, is precooked so that it swells and begins to thicken instantly when added to cold water. One particularly suitable pregelatinized starch is prepared from modified, stabilized and waxy, maize food starch, and commercially available from National Starch Company as INSTANT STARCH, ULTRASPERSE-M. Microcrystalline cellulose is another useful swelling agent.

In certain preferred embodiments of the present invention, the combined use of a structuring agent and a swelling agent as a blended thickening component is an important feature for achieving the desired liquid suspension. The use of xanthan gum with a pregelatinized starch has been found to be a particularly advantageous combination.

The nucleation inhibitor compound or compounds affects the rates of nucleation and growth, depending upon the nature of the surfaces and the structures of the adsorbed molecules. The degree of inhibition varies with the driving force for crystallization, and the mechanisms of the reactions may also be different in the presence of additives. Polyvinylpyrrolidone, also known as PVP. Polyvidone and Povidone has been found to be a particularly advantageous nucleation inhibitor.

PVP is believed, without intending to be bound by theory, to reduce the rate of sedimentation by preventing the growth of pharmaceutical particles. Ostwalt ripening provides for the growth of large particles at the expense of small ones. This effect is due to a difference in the solubility rate of the different size particles. Since the solution rate of the smaller nucleated particles is greater than that of the large crystals, dissolution of smaller particles creates a metastable state of saturation and causes eventual growth from solution onto the edge of large particles until more thermodynamically stable distribution of particle sizes is achieved. In the case of the formulations described herein, it has been found that PVP reduces the growth rates and rate of increase in particle size. The effects are especially significant for formulations that must be subjected to repeated freeze/thaw temperature cycling.

Emulsifying agents and surfactants can also be employed in the suspension compositions of the present invention, to aid in wetting the suspended particles. Suitable emulsifying agents and suspending agents include any food grade materials, such as mono and diglycerides, TWEENS and SPANS, lecithin, polyglycerol esters, propylene glycol esters and the like, Polysorbates, mono and diglycerides of fatty acids, sucrose fatty acid esters and polyoxyethylene derivatives of sorbitan fatty acid esters. These surfactants are well known in the art and are commercially available.

Suitable polyglycerol esters include triglyceryl monostearate, hexaglyceryl distearate, hexaglyceryl monopalmitate, hexaglyceryl dipalmitate, decaglyceryl distearate, decaglyceryl monooleate, decaglyceryl dioleate, decaglycerol monopalmitate, decaglycerol dipalmitate, decaglyceryl monostearate, octaglycerol monooleate, octaglycerol monostearate and decaglycerol monocaprylate.

Other useful surfactants include polysorbates made from the reaction product of monoglycerides or sorbitan esters with ethylene oxides. Examples of useful polysorbates include polyoxyethylene 20 mono- and diglycerides of saturated fatty acids, polyoxyethylene 4 sorbitan monostearate, polyoxyethylene 20 sorbitan tristearate, polyoxyethylene 20 sorbitan monooleate, polyoxyethylene 5 sorbitan monooleate, polyoxyethylene 20, sorbitan trioleate, sorbitan monopalmitate, sorbitan monolaurate, propylene glycol monolaurate, glycerol monostearate , diglycerol monostearate, glycerol lactyl-palmitate.

Other suitable surfactants include, with HLB values provided in brackets, [ ], include decaglycerol monolaurate[15.5]; decaglycerol distearate [10.5]; decaglycerol dioleate [10.5]; decaglycerol dipalmitate [11.0]; decaglycerol monostearate [13.0]; decaglycerol monooleate [13.5]; hexaglycerol monostearate [12.0]; hexaglycerol monooleate [10.5]; hexaglycerol monoshortening [12.0]; polyoxyethylene (20) sorbitan monolaurate [16.7]; polyoxyethylene (4) sorbitan monolaurate [13.3]; polyoxyethylene (20) sorbitan monopalmitate [15.6]; polyoxyethylene (20) sorbitan monostearate [14.9]; polyoxyethylene (20) sorbitan tristearate [10.5]; polyoxyethylene (20) sorbitan monooleate [15.0]; polyoxyethylene (5) sorbitan monooleate [10.0]; polyoxyethylene (20) sorbitan trioleate [11.0]. As is appreciated by those with skill in the art, the HLB value for a surfactant is an expression of its Hydrophile-Lipophile Balance, i.e., the balance of the size and strength of the hydrophilic (polar) and lipophilic (non-polar) groups of the surfactant.

Lactic acid derivatives include sodium stearoyl lactylate and calcium stearoyl lactylate.

A surfactant used in accordance with the invention is a sorbitan oleate ester, particularly, polyoxyethylene sorbitan monooleate also known as Polysorbate 80. Such surfactants or surface-active molecules consist of two ends or parts: a polar or ionic group at one end and a non-polar organic chain at the other end. Each part of the surfactant has an affinity for a different phase of the aqueous suspension. Once wetted by the aqueous phase, the surfactant provides stability by what is known as steric stabilization. The non-polar group adsorbs onto the non-wetting hydrophobic surface of the solid phase and the polar end extends into the aqueous phase. This dual absorption allows the suspended particles to be surrounded by water molecules and incorporated into the aqueous solution. In accordance with the present invention, a mixture of suspension stabilizing effective amounts of xanthan gum, pregelatinized starch and polyoxyethylene sorbitan monooleate stabilizes the suspension.

In one embodiment, the suspension stabilized in accordance with this invention comprises a suspending system including at least one thickener, a nucleation inhibitor, and at least one amino polycarboxylic acid compound.

In another embodiment, the suspension stabilized in accordance with this invention comprises a suspending system including a thickening component that is a blend of a structuring agent and a swelling agent and at least one amino polycarboxylic acid compound.

In another embodiment, the suspension stabilized in accordance with this invention further comprises surfactants and/or wetting agents. The suspension stabilizing effect of this invention addresses problems e.g., change in viscosity, pH, and particle size of the suspended particles, commonly observed with blended thickening systems.

In particular, one embodiment is a suspending system having a blended thickening component of xanthan gum and a pregelatinized starch. Another particular embodiment is a suspension comprising a suspending system having a blended thickening component of xanthan gum and pregelatinized starch and further comprising polyoxyethylene sorbitan monooleate. This embodiment is particularly useful for hydrophobic active ingredients such as loratadine. The suspension is stabilized with an effective amount of an amino polycarboxylic acid or salt thereof and a nucleation inhibitor. The amounts will vary relative depending on the type and amount of pharmaceutical active ingredient as well as the amount of taste modifying and sweetness desired for the pharmaceutical suspension.

The improved storage stability of the suspension of the present invention was demonstrated by decreased change in the critical properties of viscosity, pH, and particle size of the suspended particles compared to suspensions of the prior art upon storage. These properties were measured in an accelerated aging process in which samples were stored at an elevated temperature of 60°C. In addition, to assess particle size stability, the samples were subjected to a cycled temperature between -20°C and 40°C for four weeks. Periodically, samples were withdrawn and checked visually, and physical testing is performed using conventional equipment for measuring pH and viscosity and particle size.

The measurements were all taken at room temperature, which is generally less than or about 25°C. Undesirable changes in viscosity were evident during the foregoing stability testing when the viscosity dropped more than 25% using a Brookfield Viscometer with specified spindle, sample cup, speed, temperature and time. Similarly, undesirable changes in pH were evident when the pH fell below a threshold level. For loratadine, a pH level of about 3.7 is critical as that is where the active ingredient begins to increase in solubility and thus does not remain as a suspended system. Additionally, organoleptic properties, including color and taste, were evaluated for discernible changes under these same conditions.

The amino polycarboxylic acid compounds are represented by formula (I): 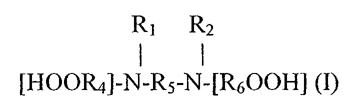 wherein R₁ and R₂, independently of one another, are hydrogen, hydroxy-terminated C₁-C₄alkylene, carboxylic-terminated C₁-C₄alkylene or N-[R₃OOH]ₘ; and R₃, R₄, R₅ and R₆ are independently of one another are C₁-C₄ alkylene and m is 1 or 2; or by formula (II): 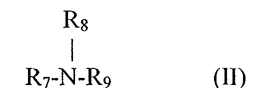 wherein R₇, R₈ and R₉, independently of one another, are hydrogen, C₁-C₄alkyl, carboxylic-terminated C₁-C₄alkylene or hydroxy-terminated C₁-C₄alkylene;
or pharmaceutically acceptable salts of formulae (I) and (II) above.

In one embodiment, at least one aminopolycarboxylic compound is represented by formula (1) wherein R₁, R₂ and R₃ are ethylene.

C₁-C₄ alkyl includes methyl, ethyl, propyl, butyl, isopropyl, sec-butyl and iso-butyl.

C₁-C₄ alkylene is methylene, ethylene, propylene, butylene, isopropylene, sec-butylene and isobutylene.

Carboxylic terminated alkylene groups are -alkylene-COOH.

Hydroxy terminated alkylene groups are -alkylene-OH.

Specific examples of amino polycarboxylic acid compounds include ethylenediaminetetraacetic acid (EDTA), hydroxyethylethylenediaminetriacetic acid, dihydroxyethylethylenediaminediacetic acid, 1,3-propanediaminetetraacetic acid, diethylenetriaminepentaacetic acid, triethylenetetraminehexaacetic acid, iminodiacetic acid, methyliminodiacetic acid, nitrilotriacetic acid, and salts thereof. They may be used as solvates such as hydrates. An embodiment uses ethylenediaminetetraacetic acid and salts thereof, specifically calcium ethylenediaminetetraacetate, disodium calcium ethylenediaminetetraacetate, sodium ethylenediaminetetraacetate, disodium ethylenediaminetetraacetate, tetrasodium ethylenediaminetetraacetate, and tetrasodium ethylenediaminetetraacetate tetrahydrate. The present invention is not limited to these examples, but one or more amino polycarboxylic acid compounds can be appropriately selected and used alone or together as a mixture.

The amount of amino polycarboxylic acid compounds to be added in compositions of the present invention depends on the nature of the compound or the dosage form of the composition. In one embodiment, in which the dosage form of the present invention is an aqueous suspension, and the polycarboxylic acid compound is disodium editate [EDTA], the level of aminopolycarboxylic acid compounds in the invention is typically from about 0.005 to about 0.1%, e.g. from about 0.02 to about 0.05% weight/volume (i.e. grams per 100 ml of suspension). In certain other embodiments, the level of polycarboxylic acid compound may be from about 0.005 to about 0.1% wt per volume (i.e. grams per 100 ml of suspension).

Taste modifying components generally comprise from about 25 to 50% by weight by volume of the total composition. The present invention however is not limited to this amount but rather to an effective amount of the taste modifying composition to produce a consumer acceptable suspension. For example, if highly intense artificial sweeteners are used a lesser amount would be required then would be the case for sugars to achieve effective taste modifying. The amount of taste modifying required would vary with the amount of pharmaceutical active ingredient used as well as the intensity of the poor taste of the pharmaceutical active ingredient. If a particular pharmaceutical active ingredient is substantially neutral in taste then the amount of taste modifying composition required could be greatly reduced.

Taste modifying compositions in accordance with the invention include but are not limited to sugars, sweet polyhydric alcohols, glycerin, artificial sweetener, flavoring agents and mixtures thereof. Examples of sugars include sucrose, fructose, dextrose, and glucose. Examples of sweet polyhydric alcohols include sorbitol and mannitol. Examples of high intensity sweeteners include aspartame, sucralose, cyclamates, asulfame K, saccharin and mixtures thereof. Examples of flavoring agents include natural and artificial fruit flavors.

Preferably a pharmaceutically acceptable organic acid, such as citric acid, is added to the suspension in a sufficient amount to adjust the pH of the solution. Other pharmaceutically acceptable organic acids are malic acid, maleic acid, tartaric acid and lactic acid.

A pharmaceutically acceptable pH adjuster, such as citric acid for acidity or sodium carbonate for alkalinity, is added to the suspension to adjust the pH of the suspension to a range between 3.7 and 8. A preferred pH range for the suspension of substantially water insoluble pharmaceutical active ingredient is between 3.8 and 5.0. Those skilled in the art understand that the use of very high levels of an organic acid, such as citric acid, will produce undesirable flavoring effects that may or may not be masked.

Preservatives useful in the present invention include, but are not limited to, benzoic acid and its pharmaceutically acceptable salts, e.g. sodium benzoate; sorbic acid and its pharmaceutically acceptable salts, e.g. potassium sorbate; and parabens (such as methyl, ethyl, propyl and butyl p-hydroxybenzoic acids esters). Preservatives, for purposes of this application, mean an antimicrobial agent. The preservatives listed above are exemplary, but each preservative must be evaluated on an empirical basis, in each formulation, to assure the compatibility and efficacy of the preservative. Methods for evaluating the efficacy of preservatives in pharmaceutical formulations are known to those skilled in the art. Sodium benzoate is presently a preferred preservative ingredient.

Preservatives are generally present in amounts of up to 1 gram per 100 mL of the suspension. Preferably the preservatives will be present in amounts in the range of from about 0.02 to about 0.5% weight by volume. For a suspension containing loratadine, the preservative sodium benzoate is present in the range of from about 0.1 to about 0.3% weight by volume. In one embodiment, sodium benzoate is present at a concentration of 0.2% weight by volume of the suspension.

Some embodiments of the present invention are summarized in the following table.

| Ingredient | Range A | Range B |
|---|---|---|
| Active Ingredient | 0.05 - 40 | |

| | Suspension System | |
|---|---|---|
| Thickener(s) | | |
| Structuring Agent | 0.1 - 0.3 | 0.15 - 0.2 |
| Swelling Agent | 0 - 3 | 1-3 |

| | Dispersants | |
|---|---|---|
| Wetting agent | 0 - 1 | 0.01 - 0.5 |
| Surfactant (30% emulsion) | 0 - 0.1 | 0.01 - 0.5 |

| | Stabilizing System | |
|---|---|---|
| Amino Polycarboxylic Acid | 0.005 - 0.1 | 0.01 - 0.05 |
| Nucleation inhibitor | 0 - 5 | 1 - 3 |

| | Suspension Modifiers | |
|---|---|---|
| Sorbitol Solution | 0-20 | 5-15 |
| Sucrose | 0-50 | 20-40 |
| Preservative | 0.02-0.5 | 0.1 - 0.3 |
| PH Modifier | 0.05 - 0.3 | 0.05 - 0.2 |
| Coloring | 0 - 0.02 | 0.005 - 0.015 |
| Sucralose Liquid Concentrate | 0-0.4 | 0.1 - 0.3 |
| Flavoring | 0-1 | 0.1 - 0.5 |

| | Properties | |
|---|---|---|
| PH | 3.7-8 | 4-6 |
| Viscosity | 700 - 1000 cps | 800 - 900 cps |

The pH and viscosity are measured within 24 hours after completing the formulation process.

The present invention also provides a process for preparing the aqueous pharmaceutical suspension composition. One inventive process comprises the following sequential steps (all units are % by weight per unit volume of the total suspension):
(a) high shear premixing from about 0.05 to 40% weight by volume of the substantially water insoluble pharmaceutical active ingredient with from about 0 to 0.1% of a surfactant, such as polyoxyethylene sorbitan monooleate and with from about 0 to 0.1% of a 30% simethicone emulsion as a defoamer;
(b) separately dispersing at least one thickener, such as xanthan gum and/or pregelatinized starch, optionally with a nucleation inhibitor, such as polyvinylpyrrolidone in about 50% water until uniformly dispersed;
(c) adding and mixing the active ingredient premix of step (a) with the aqueous mixture of step (b);
(d) adding from about 0 to 20% polyhydric alcohol sweetener, preferably sorbitol, 20 to 50% sugar, preferably sucrose, to the dispersion of step (c) and mixing until the ingredients are uniformly dispersed in the mixture;
(e) adding at least one selected preservative;
(f) adding at least one selected amino polycarboxylic acid compound and mixing to dissolution;
(g) admixing sufficient pharmaceutically acceptable pH adjuster, such as citric acid for acidity or sodium carbonate for alkalinity, to target the pH of the final solution to between about 3.7 to 8.0 to the mixture of step (f) until the ingredients are uniformly dispersed throughout the mixture;
(h) adding colorants, sweeteners, flavors, and
(i) adding and mixing sufficient water to the mixture of step (h) to produce an aqueous pharmaceutical suspension of 100% desired volume.

Another embodiment of the invention would be dry blending of a hydrophobic active ingredient with carrier in place of a high shear premix. This embodiment begins by dispersing the hydrophobic active ingredient preblend in water that contains a surfactant and ensures complete uniformity of the active ingredient in the suspension product.

In alternative embodiments of the process an effective amount of preservative such as, for example, benzoic acid, and its salts including sodium benzoate, or sorbic acid and its salts, is added to the mixture in step (e) and the suspension in step (i) is subjected to a deaerating step so that the volume of the suspension is adjusted to 100% by addition of water after such deaerating. Preferably, flavoring and coloring ingredients added to the mixture in step (h) are of the type and amount desired for the particular suspension to meet the preferences dictated by the intended consumer of such suspension, e.g. pediatric or adult. A more detailed example of the inventive process of the invention as carried out with loratadine as the active ingredient is provided in the following examples section.

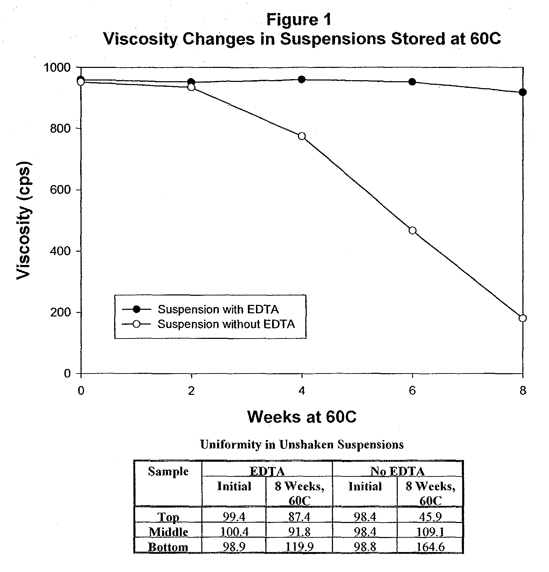

Figure 1 demonstrates the effect of EDTA addition on stabilization of suspension viscosity. Addition of EDTA stabilized suspension viscosity to maintain suspension content uniformity throughout the study period. Suspension without EDTA experienced a significant decline in viscosity resulting in loratadine sedimentation and loss of content uniformity.

Suspension viscosity was measured using a Brookfield LV Viscometer equipped with Spindle #31. Sample from an unopened bottle was dispensed into the sample chamber and equilibrated in a water bath to 25°C. After equilibration, sample was stirred at 12 rpm and viscosity read after 2 minutes.

Loratadine content of suspension was analyzed by a Gradient HPLC method at 245 nm using a Zorbax Eclipse XDB-Phenyl 4.6 x 150 mm, 3.5 µm particle size column. Mobile phase A consisted of 90:10:0.1::Water:Acetonitrile:Trifluoroacetic Acid and Mobile Phase B of 10:90:0.1::Water:Acetonitrile:Trifluoroacetic Acid. Flow rate was set at 1.0 mL/min and Injection Volume at 20 µL. To prepare samples for HPLC analysis, suspension (5 mL) was weighed into a 100 mL volumetric flask. Sample diluent (60 mL, 50:50::H₂O:ACN) was added to the flask containing suspension, shaken for 30 minutes and sample brought to volume with sample diluent.

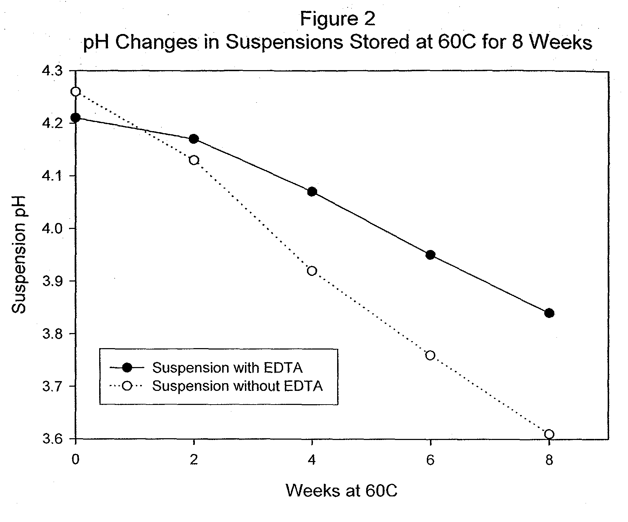

Figure 2 illustrates the effect of various pH stabilization agents on suspension pH in samples stored at 60C for 8 weeks. Samples containing EDTA exhibited improved pH stabilization compared to control samples. Suspension pH was measured using a Beckman Model 720 pH meter in accordance with USP<791>.

Tables 1 and 2 demonstrate the effect of PVP addition on particle size stability. For both accelerated and cycled temperature stability, PVP prevented loratadine crystal growth over time. In suspensions without PVP, the d90 particle size increased approximately 3 fold over the 4-week study period during accelerated and cycled temperature stability. The suspension containing 2.5% PVP showed nominal growth of loratadine crystals over the 4-week study period.

**Table 1**

| Particle Size | Suspension without PVP | | Suspension with 2.5% PVP | |
|---|---|---|---|---|
| | Initial | 4 weeks 60°C | Initial | 4 weeks 60°C |
| 50% Diameter | 8.7 | 12.2 | 9.0 | 10.2 |
| 90% Diameter | 15.6 | 75.3 | 15.9 | 22.2 |

**Table 2**

| Particle Size | Suspension without PVP | | Suspension with 2.5% PVP | |
|---|---|---|---|---|
| | Initial | 4 weeks Cycle (-20°C to 40°C) | Initial | 4 weeks Cycle (-20°C to 40°C) |
| 50% Diameter | 8.7 | 17.8 | 9.0 | 9.8 |
| 90% Diameter | 15.6 | 42.9 | 15.9 | 17.9 |

Suspension particle size was determined on a Horiba LA-910 Laser Scattering particle size distribution analyzer after starch hydrolysis using amylase. Suspension (5-mL) was aliquoted into a 100-mL volumetric flask containing 50-mL of 25 mM potassium phosphate buffer at pH 6.5. Alpha-amylase solution (10 mL of 1.28 mg/mL in 25 mM potassium phosphate buffer pH 6.5) is added to the sample solution and mixed for 3 minutes. Samples are introduced into a clean Horiba LA-91 0 set to the following instrument parameters.

| Under Conditions - Measure | |
|---|---|
| Circulation Speed: 7 | Agitation Speed: 3 |
| Ultrasonic Time: 120 (sec) | U-Sonic during Measure: No |
| Pause after U-Sonic: 10 (sec) | Sampling Times: 25 |
| Form of Distribution: Standard | Dispersant Volume: 1 x 10 (mL) |
| Dispersant Step Volume: 2 x 10 (mL) | Rinse Soln. Volume: 3 x 10 (mL) |
| Refractive Index: 1.20-0.00i | |
| Auto Conc. Adjustment: No | |

| Under Conditions - Display | | |
|---|---|---|
| Cumulative Graph: On | Type of Cumulative Graph: Undersize | |
| Scaling: Fixed | Fixed Scale: 20 | |
| Distribution Base: | Volume | % on Diameter: On 175 (micron) |
| Diameter on %: | 1 | 10(%) |
| | 2 | 30 (%) |
| | 3 | 50 (%) |
| | 4 | 70 (%) |
| | 5 | 90 (%) |

For particle size data collection, add 150-200 mL of 25 mM potassium phosphate buffer pH 6.5 to circulation well. Turn on agitation, circulation, and ultrasonic to disperse air bubbles. When the ultrasonic stops and the air bubbles are dispersed, verify laser alignment and obtain a baseline measurement. Transfer the sample into the circulation well, check the percent transmittance of the He-Ne laser is 70-95 % and collect particle size data on the sample.

The invention will now be illustrated by examples. The examples are not intended to be limiting of the scope of the present invention but read in conjunction with the detailed and general description above, provide further understanding of the present invention and an outline of a preferred process for preparing the compositions of the invention.

### EXAMPLE 1

Loratadine suspension (active 5 mg/5 ml dose) is manufactured to assess physical and chemical stability, as follows:

| Dye Premix | % w/v |
|---|---|
| Purified Water USP | 2.00 |
| Colorant | 0.013 |

| Active Ingredient Premix | |
|---|---|
| Purified Water USP | 10.0 |
| Medical Antifoam C Emulsion | 0.010 |
| Polysorbate 80 | 0.010 |
| Loratadine | 0.100 |

| Main Mix | |
|---|---|
| Purified Water USP | 50.0 |
| Pregelatinized Starch | 1.50 |
| Xanthan Gum NF | 0.180 |
| Povidone USP (Kollidon 29/32) | 2.50 |
| Sorbitol Solution USP 70% | 10.0 |
| Sucrose NF | 35.0 |
| Disodium EDTA USP | 0.025 |
| Sodium Benzoate NF | 0.200 |
| Citric Acid, anhydrous USP | 0.110 |
| Sucralose Liquid Concentrate | 0.200 |
| Flavor | 0.200 |
| Purified Water USP qs. | 100% w/v |

### Manufacturing Procedure

Dyes are solubilized in water in a separate container. A high shear active ingredient premix is processed in a separate vessel. Loratadine is dispersed in water to which has been added Polysorbate 80 and Medical Antifoam C Emulsion. This is reserved for later use in the batch. In the main mix tank equipped with a propeller or high shear mixer, water is charged and pregelatinized starch, xanthan gum and PVP are dispersed and hydrated. The Loratadine premix is added and mixed. Sorbitol solution is added followed by sucrose and mixed until dissolved. Sodium benzoate is added and mixed until dissolved. Citric acid is added followed by EDTA and mixed to dissolve followed by the dye premix, Sucralose and flavor. The batch is brought to volume.

### SUMMARY OF RESULTS:

The suspension of this example exhibited superior chemical and physical stability under stress stability conditions compared with suspensions not containing an amino carboxylic acid or a nucleation inhibitor. Additionally, the suspensions of this example exhibited acceptable taste and color stability after storage at stressed conditions.

### EXAMPLE 2

Product depicted in Figures 1 and 2 is prepared to assess physical stability, as follows:

| Dye Premix | Suspension with EDTA % w/v | Suspension without EDTA %w/v |
|---|---|---|
| Purified Water USP | 1.00 | 1.00 |
| Colorant | 0.013 | 0.013 |

| Active Ingredient Premix | | |
|---|---|---|
| Pregelatinized Starch | 1.5 | 1.5 |
| Loratadine | 0.100 | 0.100 |

| Main Mix | | |
|---|---|---|
| Purified Water USP | 60.0 | 60.0 |
| Polysorbate 80K NF | 0.010 | 0.010 |
| Xanthan Gum NF | 0.180 | 0.180 |
| Sorbitol Solution USP 70% | 10.0 | 10.0 |
| Sucrose NF | 35.0 | 35.0 |
| Disodium EDTA USP | 0.025 | 0 |
| Sodium Benzoate NF | 0.200 | 0.200 |
| Citric Acid, anhydrous USP | 0.110 | 0.110 |
| Sucralose 25% Liquid Concentrate | 0.200 | 0.200 |
| Flavor | 0.200 | 0.200 |
| Purified Water USP qs. | 100% w/v | 100% w/v |

### Manufacturing Procedure

Dyes are solubilized in water in a separate container. A high shear active ingredient premix is processed in a separate vessel. Loratadine is dispersed in water to which has been added Polysorbate 80 and Simethicone Emulsion. This is reserved for later use in the batch. In the main mix tank equipped with a propeller or high shear mixer, water is charged and pregelatinized starch, xanthan gum and PVP are dispersed and hydrated. The Loratadine premix is added and mixed. Sorbitol solution is added followed by sucrose and mixed until dissolved. Sodium benzoate is added and mixed until dissolved. Citric acid is added followed by EDTA and mixed to dissolve followed by the dye premix, Sucralose and flavor. The batch is brought to volume.

Suspensions with and without EDTA prepared according to this procedure are packaged in high density polyethylene (HDPE) bottles, and stored at 60 C for 8 weeks, with bottles being removed for testing at 2-week intervals. Samples were tested for viscosity and pH according to methods described previously herein, with results shown in Figures 1 and 2 respectively. Results indicate the addition of EDTA in the formula has a stabilizing effect on both viscosity and pH.

## Claims

1. A pharmaceutical aqueous suspension comprising:
a) a therapeutically effective amount of suspended solid particles comprising at least one active ingredient;
b) a thickener;
c) a nucleation inhibitor; and
d) at least one amino polycarboxylic acid compound; and
wherein the suspension has a pH of about 3.7 to 8.

2. A suspension according to claim 1, wherein the suspended solid particles are hydrophobic and the suspension further comprises a surfactant.

3. A suspension according to claim 1, wherein the suspended solid particles have a median particle size, as measured by laser scattering, of about 1 to about 20 microns.

4. A suspension according to claim 1, wherein the active ingredient is substantially insoluble in an aqueous environment at room temperature.

5. A suspension according to claim 1 wherein the viscosity remains constant for at least two weeks when stored at a temperature of at least 60°C.

6. A suspension according to claim 1 wherein the amino polycarboxylic acid compound is a compound according to formula (I) and pharmaceutically acceptable salts thereof: 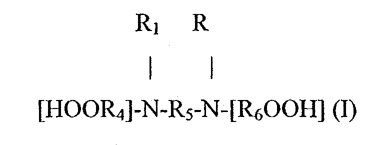 wherein R₁ and R₂, independently of one another, are hydrogen, hydroxy-terminated C₁-C₄ alkylene, carboxylic-terminated C₁-C₄ alkylene or N-[R₃OOH]ₘ;
and R₃, R₄, R₅ and R₆ are independently of one another are C₁-C₄ alkylene
and m is 1 or 2;
or formula (II) 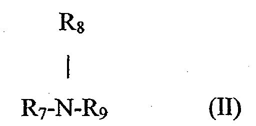 wherein R₇, R₈ and R₉, independently of one another, are hydrogen, C₁-C₄ alkyl, carboxylic-terminated C₁-C₄ alkylene or hydroxy-terminated C₁-C₄ alkylene
and pharmaceutically acceptable salts of formula (I) or (II).

7. A suspension according to claim 1, wherein the amino polycarboxylic acid compound is disodium ethylenediaminetetraacetate.

8. A suspension according to claim 1 wherein the active ingredient is loratadine.

9. A pharmaceutical aqueous suspension comprising:
a) a therapeutically effective amount of suspended solid particles comprising at least one active ingredient;
b) a blended thickening component, said thickening component comprising a swelling agent and a structuring agent;
c) at least one amino polycarboxylic acid compound; and
wherein the suspension has a pH of about 3.7 to 8.

10. A pharmaceutical aqueous suspension comprising a therapeutically effective amount of suspended solid particles comprising at least one active ingredient selected from the group consisting of fexofenadine, loratadine, desloratadine, terfenadine, astemizole, norastemizole, cetirizine, and pharmaceutically acceptable salts, esters, isomers, and mixtures thereof,
wherein the suspension has a pH of about 3.7 to 8; and
wherein the suspended solid particles have a median particle size, as measured by laser scattering, of about 1 to about 20 microns after 4 weeks at 60°C.
